(19) Europäisches Patentamt
European Patent Office
Office européen des brevets

(11) Publication number: **0 148 007**
**A2**

(12) # EUROPEAN PATENT APPLICATION

(21) Application number: 84308969.9

(22) Date of filing: 20.12.84

(51) Int. Cl.[4]: C 07 D 501/20
A 61 K 31/545

(30) Priority: 26.12.83 JP 248929/83
07.03.84 JP 41992/84

(43) Date of publication of application:
10.07.85 Bulletin 85/28

(84) Designated Contracting States:
DE FR GB IT

(71) Applicant: YAMANOUCHI PHARMACEUTICAL CO. LTD.
No. 5-1 Nihonbashi-Honcho, 2-chome Chuo-ku
Tokyo(JP)

(72) Inventor: Nagano, Noriaki
3-24-13, Asamadai
Ageo-shi Saitama(JP)

(72) Inventor: Hara, Ryuichiro
3-9-11, Honcho
Nakano-ku Tokyo(JP)

(72) Inventor: Murakami, Yukiyasu
306-3, Ohmaki
Urawa-shi Saitama(JP)

(72) Inventor: Nakano, Kohji
1086-1, Ohaza Sanegaya Shiraoka-cho
Minamisaitama-gun Saitama(JP)

(72) Inventor: Kouda, Akio
4-5-3,Hibarigaoka Kita
Hohya-shi Tokyo(JP)

(72) Inventor: Maeda, Tetsuya
3-14-6, Yotsuya
Urawa-shi Saitama(JP)

(72) Inventor: Shibanuma, Tadao
596-11, Ohyaguchi
Urawa-shi Saitama(JP)

(72) Inventor: Yamazaki, Atsuki
2-7-8-506, Shimura
Itabashi-ku Tokyo(JP)

(72) Inventor: Matsui, Hidefumi
227-53, Kitamotojuku
Kitamoto-shi Saitama(JP)

(74) Representative: Geering, Keith Edwin et al,
REDDIE & GROSE 16 Theobalds Road
London WC1X 8PL(GB)

(54) **Cephalosporin compounds and medicaments and their production.**

(57) Antibacterial compounds of formula (I),

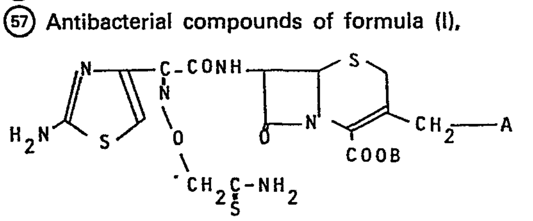

(I)

wherein

A represents a hydrogen atom, a carbamoyloxy group, (4-carboxy-3-hydroxytisothiazol-5-yl) thio group

(that is, 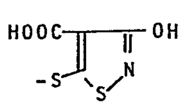 ),

or a group shown by the formula :

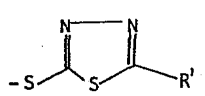

(wherein R' is a lower alkyl group); B represents a hydrogen atom

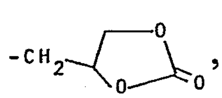,

$-CH_2OOC-C(R^2)_3$ or a pharmaceutically acceptable cation for forming carboxylate salt; and $R^2$ is a lower alkyl group.

EP 0 148 007 A2

0148007

# CEPHALOSPORIN COMPOUNDS AND MEDICAMENTS AND THEIR PRODUCTION

This invention relates to antibacterial cephalosporin compounds which are especially useful for oral administration, the production of these compounds, medical compositions containing them, and salts thereof.

The compounds of this invention are those of the general formula (I) and salts thereof:

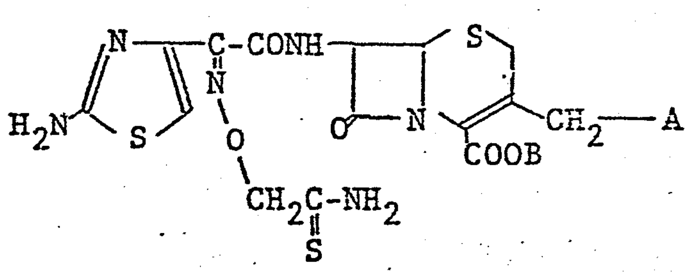

(I)

wherein A represents a hydrogen atom, a carbamoyloxy group, (4-carboxy-3-hydroxyisothiazol-5-yl)thio group (that is, 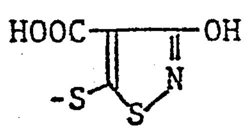 ),

or a group shown by the formula:

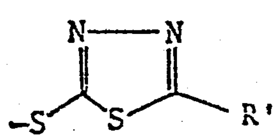

(wherin R' is a lower alkyl group);

B represents a hydrogen atom, 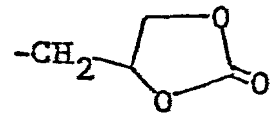 ,

$-CH_2-OOC-C(R^2)_3$ (where $R^2$ is a lower alkyl group) or a cation forming a pharmaceutically acceptable carboxylate salt.

Many 3-substituted cephalosporin compounds having a substituted oxyimino moiety in the 7-position substituent are known. Particularly, German Offenlegungschrift No. 2912829 discloses cephalosporin compounds having oxyimino moiety substituted by a thiocarbamoylmethyl group. That is, there is generically disclosed in this German publication, cephalosporin compounds of the formula

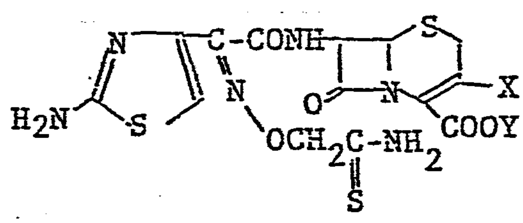

wherein X is a $C_1$ to $C_5$ alkyl group, a carbamoyloxymethyl group or $-CH_2S-Z$ (Z is a heterocyclic group containing a nitrogen atom), etc., Y is a hydrogen atom, an alkali metal atom, or other groups for forming a carboxyl ester.

However, compounds of the present invention are not concretely disclosed in the above German publication, which also does not disclose that its compounds have utility for oral administration.

Absorbability by the intestinal tract membrane is not good in the case of known cephalosporin compounds, and the known cephalosporin compounds have usually been administered by injection. Thus the purpose of the present invention is to provide novel cephalosporin compounds which can be administered orally.

The substituent at the 4-position of the cephalosporin nucleus of the compounds of this invention is a free carboxyl group (or salt thereof) or a carboxy ester group such as $-COOCH_2OOCC(R^2)_3$,

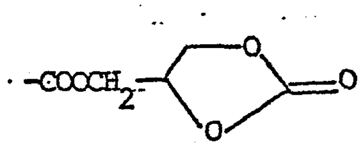

(-COO-pivaloyloxymethyl is especially preferred). Even the compounds of the present invention having a free carboxyl group at the 4-position have good asbsorbability by the intestinal tract membrane and excellent antibacterial activity to produce the desired therapeutic effect on oral administration. Compounds of the present invention in which the substituent at the 4-position is a carboxy ester group also have good absorbability by the intestinal tract membrane. In the case of such ester compounds, the ester moiety at the 4-position is converted to a free carboxyl group by hydrolysis with intracellular enzyme. Thus, there

is provided a high blood concentration of the free carboxyl compound when a compound of formula (I) is administered orally, and the high blood concentration is maintained for a long time.

The above-mentioned compounds (I), and below-mentioned compounds ($I_a$) and ($I_b$) and starting compounds (II) include geometrical isomers and tautomers; this invention includes all these syn-form and anti-form geometrical isomers and mutual tautomers. The syn form compounds (I) have a chemical structure portion represented by

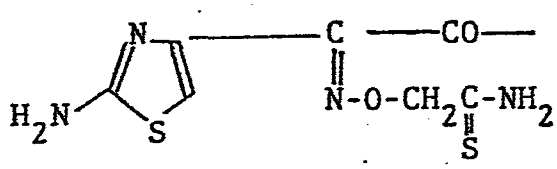

and the anti form compounds (I) have a chemical structure portion represented by

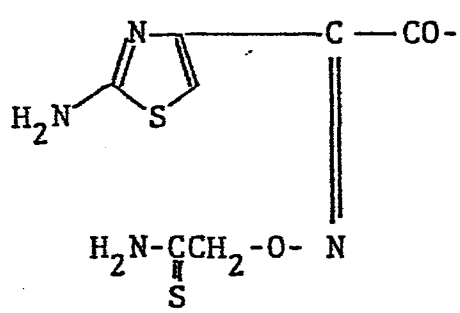

The salts of the compounds (I) include pharmaceutically acceptable nontoxic salts or pharmacologically acceptable salts. Examples of such salts are the usual nontoxic salts, for example, salts of inorganic bases (e.g. of an alkali metal such as sodium or potassium or of an alkaline earth metal such as calcium or magnesium); ammonium salts; salts of organic bases such as trimethylamine, triethylamine, pyridine, picoline,

cyclohexylamine, dicyclohexylamine, diethanolamine, N,N'-dibenzylethylenediamine; salts of organic acids such as acetic acid, trifluoroacetic acid, lactic acid, tartaric acid, fumaric acid, maleic acid, methanesulfonic acid, ethanesulfonic acid, benzenesulfonic acid, formic acid, toluenesulfonic acid; salts of inorganic acids such as hydrochloric acid, sulfuric acid, phosphoric acid, hydrobromic acid; and salts of amino acids such as arginine and lysine, asparaginic acid, glutamic acid.

The term "lower" in the definitions herein means a straight or branched carbon chain having 1 to 4 carbon atoms. Thus examples of the "lower alkyl" are methyl, ethyl, n-propyl, iso-propyl, n-butyl, iso-butyl, and tert-butyl.

The compouds of this invention can be produced by various processes; typical production processes are explained hereinafter:

Process 1:

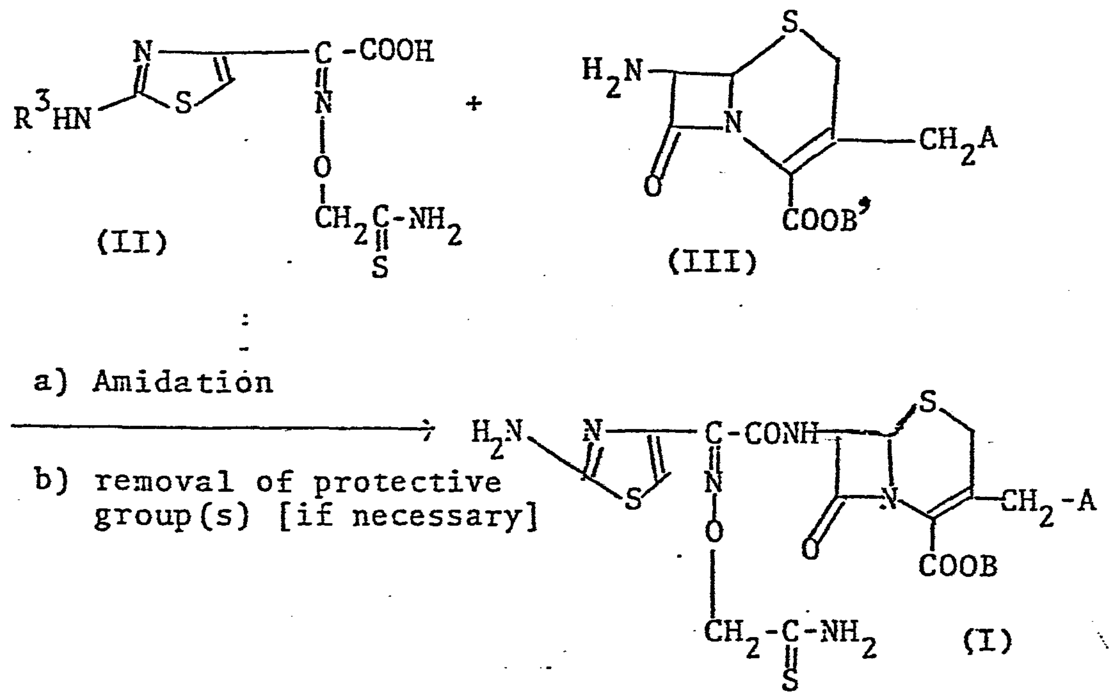


(II) + (III) → (I)

In the above formulae, A and B are as defined above, $R^3$ is a hydrogen atom or a protective group for an amino group, B' is a hydrogen atom, a pharmaceutically acceptable cation, a protective group for a carboxyl group,

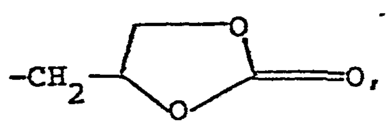, $-CH_2OOCC(R^2)_3$ (wherein $R^2$ is a lower alkyl). Symbols herein have the same significances throughout unless otherwise indicated.

Compounds I can thus be produced by reacting substituted oxyiminothiazolylacetic acid derivative II or reactive derivative thereof with 7-amino-3-cephem derivative III and then, if necessary, releasing any protective group(s).

In this case the protective group for an amino group may be one usually used in the field of peptide chemistry and practical examples are acyl groups such as a formyl group, an acetyl group, a propionyl group, a tert-butoxycarbonyl group, a methoxyacetyl group, a methoxypropionyl group, a benzyloxycarbonyl group, a p-nitrobenzyloxycarbonyl group; tri-loweralkylsilyl groups such as a trimethylsilyl group; and aralkyl groups such as a benzyl group, a benzhydryl group (diphenylmethyl group), a trityl group.

Practical examples of the protective group for a carboxy group are those which can be released easily under mild conditions, such as a benzhydryl group, a $\beta$-methylsulfonylethyl group, a phenacyl group, a p-methoxybenzyl group, a tert-butyl group, a p-nitrobenzyl group; and lower-alkylsilyl groups such as trimethylsilyl group.

The reaction is usually performed in a solvent under cooling at room temperature or below. Any

solvents which do not take part in the reaction can be used. Examples of the solvent usually used are organic solvents such as dioxane, tetrahydrofuran, ether, acetone, ethyl methyl ketone, chloroform, dichloromethane, dichloroethane, methanol, ethanol, acetonitrile, ethyl acetate, ethyl formate, dimethylformamide, dimethyl sulfoxide; these solvents may be used alone or in appropriate combination.

A compound II may be a free carboxylic acid or a reactive derivative thereof. Suitable examples of the compound are mixed acid anhydrides, acid anhydrides, acid halides, active esters, active amides, acid azides. When using the compound in the form of a free carboxylic acid, it is preferred to use a condensing agent such as N,N'-dicyclo-hexylcarbodiimide or N,N'-diethylcarbodiimide.

According to the kind of reactive derivative of carboxylic acid used, it may be preferred for smooth reaction to operate in the presence of a base. Examples of such a base are inorganic bases such as sodium hydrogencarbonate, potassium hydrogencarbonate, sodium carbonate, potassium carbonate; and organic bases such as trimethylamine, triethylamine, dimethylaniline, pyridine.

The removal of an amino protecting group from the reaction product thus obtained is easily performed,

when the group is an aralkyl group (e.g. trityl) or an acyl group, by hydrolysis with acid; and when the group is tri-loweralkylsilyl such as trimethylsilyl, by contact with water. As the acid used in this case, formic acid, trifluoroacetic acid, hydrochloric acid, are amongst those preferred. Removal of a carboxy-protecting group is easily performed using an acid in the case of a benzhydryl group, a tert-butyl group, or a p-methoxybenzyl group, or by contact with water in the case of tri-loweralkylsilyl such as trimethylsilyl group. Removal of carboxy- and amino-protecting groups can be performed simultaneously.

A salt of the formula I compound can be produced by performing the foregoing production process using a salt of the starting compound, or by applying a salt-forming reaction to the free formula I compound. In the latter case, for example, an alkali metal salt can be produced by adding an n-butanol solution of an alkali 2-ethylhexanoate to the reaction product and then adding thereto an organic solvent having different solubility, such as ether or ethyl acetate; a salt of an organic base or a basic amino acid can be obtained by adding an equivalent amount or a slight excess of organic base or basic amino acid such as dicyclohexylamine, triethylamine, cyclohexylamine, diethanolamine, arginine or lysine to the reaction product; and an ammonium

salt can be obtained by adding aqueous ammonia to the reaction product.

The formula I compounds and salts can be separated and purified in ordinary manner, such as extraction with organic solvent, crystallization, column chromatography.

Process 2:

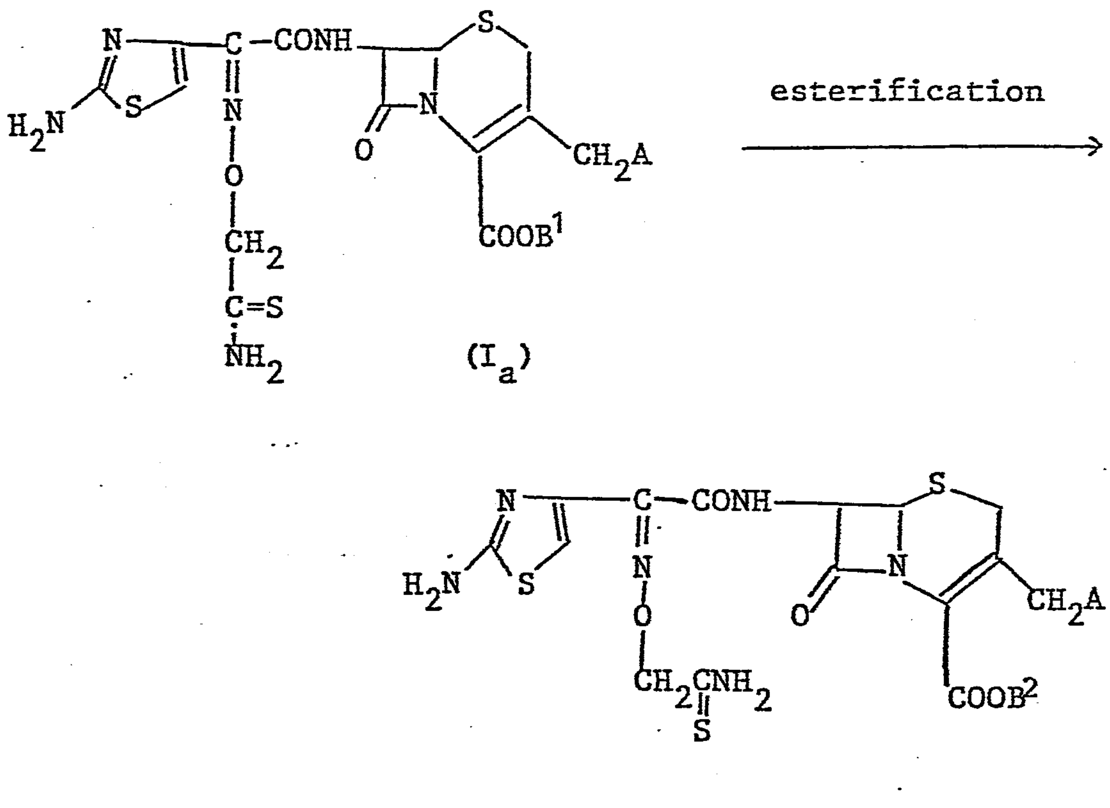


$(I_a)$

$(I_b)$

In the above formulas, $B^1$ represents a hydrogen atom or a (pharmaceutically acceptable) cation for forming a salt with carboxy anion ; $B^2$ represents $-CH_2OCOC(R^2)_3$ or 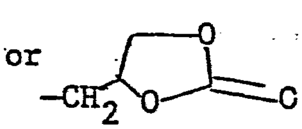 ; and $R^2$ represents a lower alkyl group.

Compounds $I_b$ or salts thereof can be produced by applying an ester-forming reaction to the formula $I_a$ compound. The salts of the compounds $I_b$ include pharmaceutically acceptable salts, examples of which are the same as those for salts of the compounds I.

The ester-forming reaction is performed by reacting the compound $I_a$ with an esterification agent. Examples of such an esterification agent are alcohol, alkyl halide, alkyl sulfonate, alkyl sulfate and diazocompound; any equivalent agent for the esterification can be used.

The reaction is usually performed in a solvent. Any solvents which do not take part in the reaction can be used. Examples of the solvent usually used are dimethylformamide, tetrahydrofuran, dioxane, dimethylsulfoxide.

The reaction is usually performed under cooling or under mild heating. A compound $I_a$ may be a free carboxylic acid or a reactive derivative thereof. When using the compound in the form of a free carboxylic acid, it is preferred to operate in the presence of a base. Examples of such a base are inorganic bases

such as alkali metal (for example, sodium, potassium, etc.), alkali earth metal (for example, magnesium, calcium , etc.), hydroxide of such metal, carbonate of such metal, hydrogencarbonate of such metal; and organic bases such as alkali metal alkoxide (for example, sodium methoxide, sodium ethoxide, etc.), alkali metal alcanoate (for example, sodium acetate), trialkylamine (for example, triethylamine), pyridine compounds (for example, pyridine, lutidine, picoline), quinoline.

Compounds of this invention have shown antibacterial activity against various pathogens including gram positive and negative pathogens, and are useful for medicaments (especially as antibacterial agents), additives for feeds, and preservatives. Compounds of this invention are especially useful for oral administration.

Antibacterial medicaments containing compounds according to the invention may be prepared by conventional methods using conventional carriers or excipients - such carriers or excipients may be organic or inorganic, solid or liquid material which are pharmaceutically acceptable and suitable for oral administration or parenterally. They may for example be administered orally as tablets, pills, capsules, granules; parenterally by intravenous or intramuscular injection; or as suppositories.

Among such administration methods, administration by the oral route is suitable for the compounds of the present invention.

The appropriate dose is determined in each case considering factors such as the symptom, age and sex of the patient, but for an adult a daily total of 250 to 3,000 mg is usually administered in one to four doses.

Antibacterial activities (minimum effective inhibitory concentrations) are illustrated below by the activity demonstrated by one of the preferred compound.

Test compound: 7-[α-(2-amino-4-thiazolyl)-α-(thiocarbamoylmethoxyimino)acetamido]-3-methyl-$\Delta^3$-cephem-4-carboxylic acid. (syn–form)

Test method: Heart infusion agar disc method.

Result:

Table (Minimum Effective Inhibitory Concentrations; μg/ml)

| Strain \ The test compound : as above | |
|---|---|
| Staph. aureus ATCC 6538p | 1.56 |
| E. coli NIHJ | 0.05 |
| Kleb. pneumoniae ATCC 10031 | 0.05 |
| E. coli Ebara | 3.13 |
| Serr. marcescens NY-10 | 0.78 |

In addition, test data about absorbability of a preferred compound are shown below.

Test compound: 7-[α-(2-amino-4-thiazolyl)-α-(thiocarbamoylmethoxyimino)acetamido]-3-methyl-$\Delta^3$-cephem-4-carboxylic acid (syn form); Test dosage: 50 mg /kg.; Test animals: rats.

Result:

The proportion (%) of the test compound excreted in urine during 0-24 hours after oral administration was 11.7 %

The proportion (%) of the test compound excreted in bile during 0-24 hours after oral administration was 9.8 %.

0148007

Liquid ratios herein are by volume unless otherwise indicated.

Reference Example 1

i)

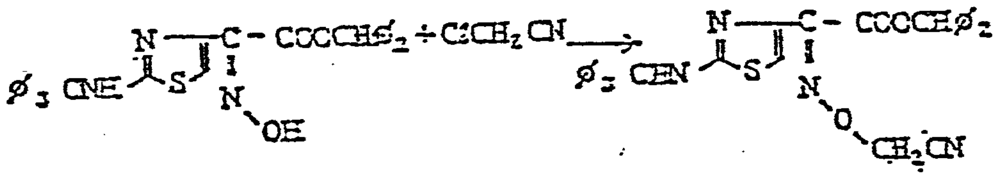

In 70 ml of dimethylsulfoxide was dissolved 11.9 g (0.02 mole) of (Z)-2-(2-tritylamino-4-thiazolyl)-2-(hydroxyimino)acetic acid benzhydryl ester and after adding thereto 2.76 g of a powder of potassium carbonate and 1.7 g (0.0225 mole) of chloroacetonitrile, the mixture was allowed to react overnight at room temperature. After the reaction was over, the reaction mixture was poured into 150 ml of ice water, the product was extracted first with 150 ml of ethyl acetate and then with 100 ml of ethyl acetate. The organic layers were collected, and washed twice with 50 ml of water and then with 50 ml of a saturated aqueous sodium chloride solution. The ethyl acetate layer obtained was dried over anhydrous magnesium sulfate, and the solvent was distilled off to provide a caramel. To the caramel was added 100 ml of ethyl ether to powder the caramel. 11.5 g (yield: 90.7 %) of (Z)-2-(cyanomethyloxyimino)-2-(2-tritylamino-4-thiazolyl)acetic acid benzhydryl ester was obtained.

IR $\nu_{max}^{KBr}$ $cm^{-1}$;

3330, 1730, 1525, 1245, 1165, 1015, 735, 690

NMR ($CDCl_3$)

$\delta$ (ppm);

4.72 (1H, s, $-CH_2-$ )

6.33 (1H, s, 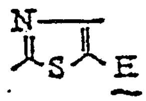 )

6.85 (1H, s, $-CH\phi_2$ )

7.2 ~ 7.3 (25H, 5$\phi$)

ii)

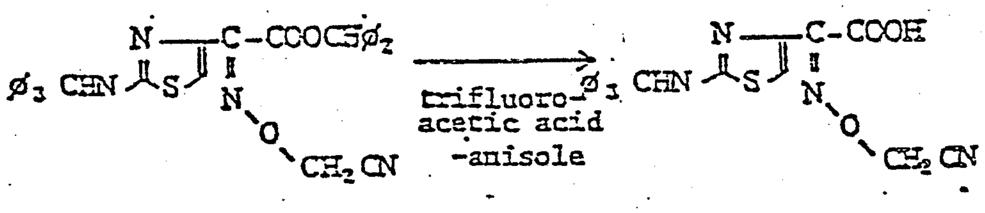

11.3 g (17.8 millimole) of the foregoing compound was dissolved in 26 ml of dichloromethane and 6.5 ml of anisole, and the solution was cooled to -30°C. 19 ml of trifluoroacetic acid was added dropwise to the solution below -20°C. Thereafter, the reaction proceeded for 1 hour at -19 ~ -21°C. The reaction solution was distilled off under reduced pressure at low temperature. To the oily residue thus formed was added 150 ml of ether-n-hexane (1:3) to powder the residue. 6.6 g of (Z)-2-(cyanomethyl-oxyimino)-2-(2-tritylamino-4-thiazolyl) acetic acid was obtained. IR $\nu_{max}^{KBr}$ $cm^{-1}$ ;

2900~3100, 1590, 1565, 1190, 695

NMR ($d_6$-DMSO)

δ (ppm) ;

5.05 (2H, s, $-\underset{\sim}{CH_2}-$ )

7.04 (1H, s, 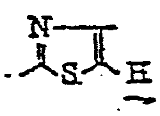 )

7.2~7.5 (15H, 3ø)

8.94 (1H, s, $-\underset{\sim}{NH}-$ )

iii)

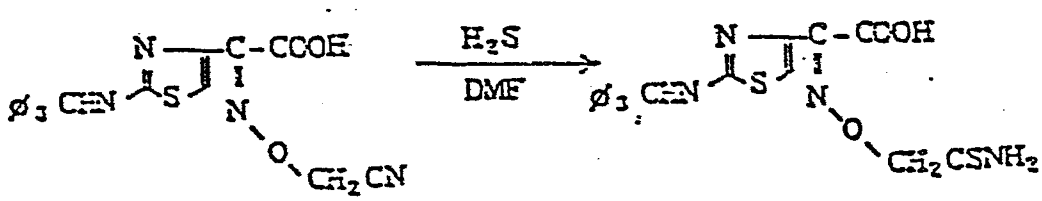

In 20 ml of N,N-dimethylformamide was dissolved 2.814 g (6 millimole) of the foregoing compound and after adding thereto 1.6 ml of triethylamine, the mixture was reacted for 1 hour at 40-45°C while passing hydrogen sulfide gas therethrough. After the reaction was over, hydrogen sufide was removed by passing nitrogen gas through the reaction mixture at room temperature. The resultant mixture was poured into a solution containing 100 ml of water and 15 ml of 1N-hydrochloric acid solution to form a white powder. The powder was collected by filtration, washed with water, and dried to provide 2.11 g of (Z)-2-(thiocarbamoylmethoxyimino)-2-(2-tritylamino-4-thiazolyl)acetic acid.

IR $\nu_{max}^{KBr}$ $cm^{-1}$ ;

3000~3050, 1560~1590, 1440, 1370, 1265, 1185, 1050, 1030, 965, 830, 730, 695

NMR ($d_6$-DMSO)

4.78 (2H, s, $-CH_2-$)

6.98 (1H, s, 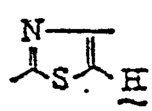)

7.2~7.5 (15H, 3ø)

Reference Example 2

i)

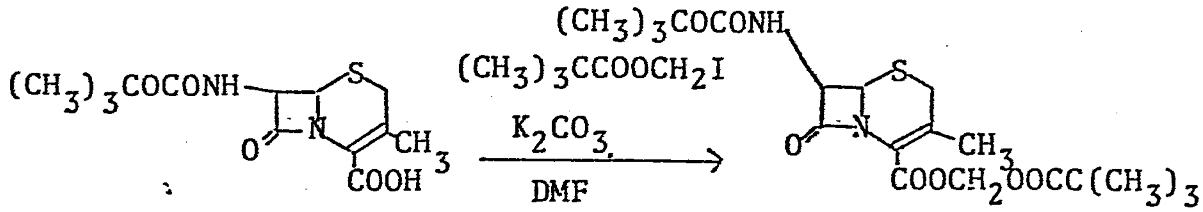


To 15 ml of dimethylformamide were added under ice-cooling 3.14 g of 7-(tert -butoxycarbonyl)amino-3-methyl-$\Delta^3$-cephem-4-carboxylic acid and 0.83 g of potassium carbonate and after adding thereto 2.90 g of pivaloyloxymethyl iodide, the mixture was stirred for 2 hours at 0-2°C. To the reaction mixture was added 150 ml of ethyl acetate and after washing the mixture three times each time with 30 ml of water and then with 30 ml of saturated aqueous sodium chloride, the mixture was dried over magnesium sulfate and concentrated under reduced pressure to remove ethyl acetate. The residue was subjected to column chromatography on silica gel and the product was eluted with a mixture of ethyl acetate and benzene (volume ratio of ethyl acetate benzene 1:10). The fractions containing the desired product were collected and concentrated to provide 3.4 g pivaloyloxymethyl 7-(tert-butoxycarbonyl)amino-3-methyl $\Delta^3$-cephem-4-carboxylate.

IR $\nu_{max}^{KBr}$ $cm^{-1}$: 3300,2950,1780,1740,1710,1630, 1520,1360,1150

NMR($CDCl_3$-TMS): $\delta$(ppm);

1.20 (9H, s, t-Butyl)

1.44 (9H, s, t-Butyl)

2.12 (3H, s, $CH_3$-)

3.36 (2H, q, 2: $-CH_2-$)

4.96 (1H, d, 6: -C<u>H</u>-)

5.56 (1H, q, 7:-CH-)

5.90 (2H, q, -COOC<u>H</u>$_2$O-)

FABMS; $(M+1)^+$=429

ii)

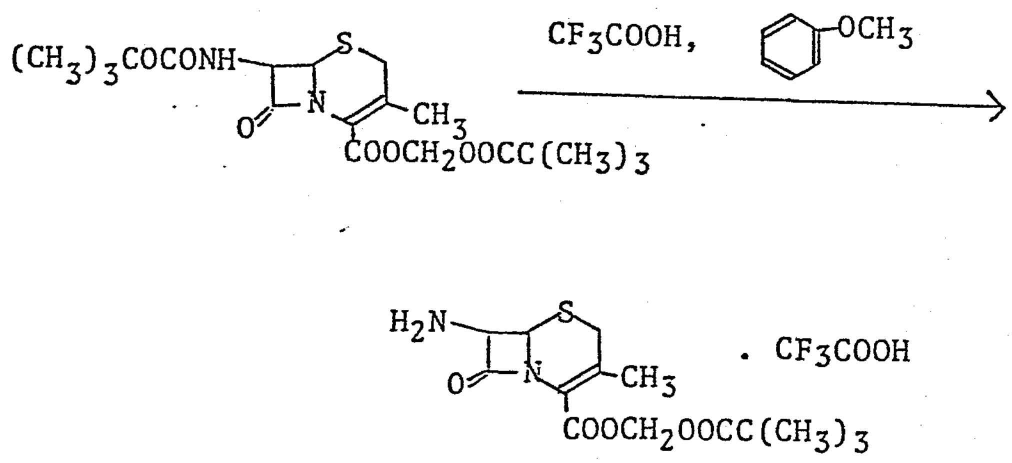


To 1.28 g of pivaloyloxymethyl 7-(tert-butoxycarbonyl)amino-3-methyl-$\Delta^3$-cephem-4-carboxylate was added 1.2 ml of anisole to form a paste. After adding thereto 6.0 ml of trifluoroacetic acid, the mixture was stirred for 1 hour at room temperature. The reaction solution was concentrated under reduced pressure, and the residue was subjected to silica gel column chromatography. The product was eluted with ethyl acetate, and the fractions

containing the desired product were collected and concentrated to provide 1.08 g of pivaloyloxymethyl 7-amino-3-methyl-$\Delta^3$-cephem-4-carboxylate trifluoroacetic acid salt.

IR $\nu_{max}^{KBr}$ $cm^{-1}$;

2950, 1780, 1740, 1660, 1380, 1195, 1125.

NMR ($CDCl_3$-TMS)

δ(ppm); 1.20 (9H, s, t-Butyl)

2.24 (3H, s, $-CH_3$)

3.36 (2H, q, 2-position: $-C\underline{H}_2-$)

4.96 (1H, d, 6-position: $-C\underline{H}-$)

5.10 (1H, d, 7-position: $-C\underline{H}-$)

5.86 (2H, q, $COOC\underline{H}_2O-$)

Reference Example 3

i)

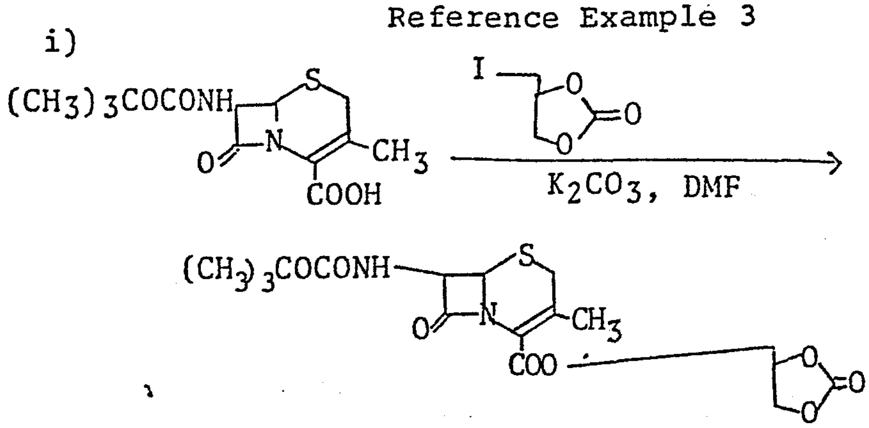


To 10 ml of dimethylformamide were added 3.14 g of 7-(tert-butoxycarbonyl)amino-3-methyl-$\Delta^3$-cephem-4-carboxylic acid and 759 mg of potassium carbonate, and the mixture was stirred under ice-cooling. After adding thereto 2.50 g of 2-oxo-4-iodomethyl-1,3-dioxolan, the mixture was stirred at room temperature overnight. To the reaction mixture was added 100 ml of ethyl acetate, and the mixture was washed three times each time with 30 ml of water. The wash liquid (separated aqueous layer) was extracted once with 30 ml of ethyl acetate and the organic layers were combined, washed three times each time with 20 ml of saturated aqueous sodium chloride solution and dried over magnesium sulfate. The solvent was distilled off under reduced pressure to provide 3.3 g of crude product. The crude product was subjected to column chromatography on silica gel. The product was eluted with a mixture of ethyl acetate and benzene in a ratio of 1:5. The fractions containing the desired product were collected and concentrated to provide 1.02 g of 2-oxo-1,3-dioxolan-4-ylmethyl 7-(tert-butoxycarbonyl)amino-3-methyl-$\Delta^3$-cephem-4-carboxylate.

IR $\nu_{max}^{KBr}$ $cm^{-1}$;

3400, 2950, 1770, 1710, 1510, 1360, 1150

NMR ($CDCl_3$-TMS)

δ(ppm);

1.44 (9H, s, tert-Butyl)

2.16 (3H, s, $-CH_3$)

3.40 (2H, q, 2: $-CH_2-$)

3.3~3.6 (4H, m, 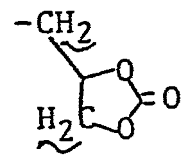

4.9~5.1 (1H, m, 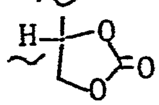

4.96 (1H, d, 6: -CH-)

5.48 (1H, q, 7: -CH-)

5.4~5.6 (1H, bs, -CONH-)

FABMS; $(M+H)^+ = 415$

ii)

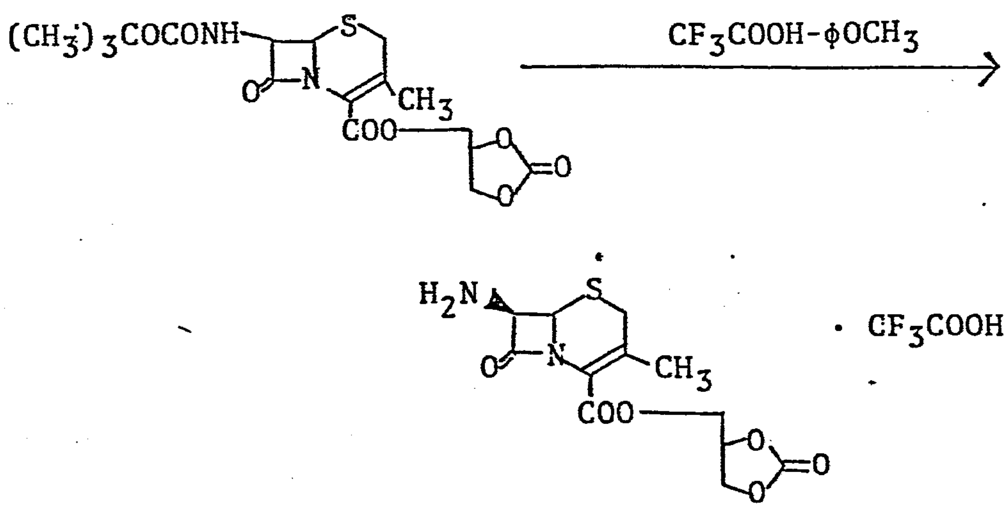

To 850 mg of 2-oxo-1,3-dioxolan-4-ylmethyl 7-(tert-butoxycarbonyl)amino-3-methyl-$\Delta^3$-cephem-4-carboxylate were added 1 ml of anisole and 5 ml of trifluoroacetic acid, and the mixture was stirred for 1 hour at room temperature. The reaction mixture was concentrated under reduced pressure in order to remove solvent. To the residue was added 10 ml of ether, and the formed powder was collected by filtration to provide 588 mg of trifluoroacetic acid salt of 2-oxo-1,3-dioxolan-4-ylmethyl 7-amino-3-methyl-$\Delta^3$-cephem-4-carboxylate as a hygroscopic powder.

IR $\nu_{max}^{KBr}$ $cm^{-1}$;

3400, 1770, 1720, 1670, 1380, 1160, 1040

NMR (DMSO-$d^6$/TMS)

δ(ppm);

2.12 (3H, s, $-C\underline{H}_3$)

3.4～3.6 (2H, m, 2: $-C\underline{H}_2-$)

4.2～4.6 (4H, m, 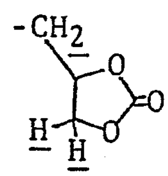

4.9～5.2 (1H, m, 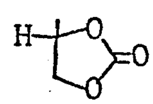

5.10 (1H, d, 6: $-C\underline{H}-$)

5.22 (1H, d, 7: $-C\underline{H}-$)

Reference Example 4

(i)

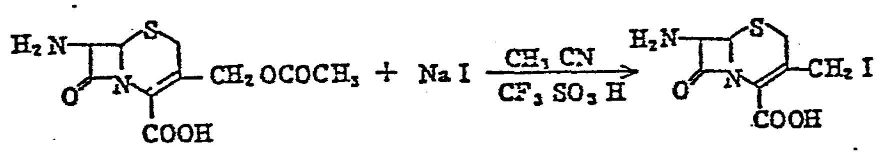


To a mixture of 2.5 g (0.0092 mole) of 3-acetoxymethyl-7-amino-$\Delta^3$-cephem-4-carboxylic acid, 1.52 g of sodium iodide and 12.5 ml of acetonitrile was added 5 ml of trifluoromethylsulfonic acid dropwise over a period of 7 minutes while cooling the mixture to 18°C. The reaction proceeded at 12-18°C for a period of 17 minutes; after pouring into 50 ml of ice-water, the resulting mixture was stirred for 20 minutes under ice-cooling, and the crystals formed were collected by filtration. The crystals thus obtained were washed with 32.5 ml of ice-water and then with 25 ml of acetone, and dried with phosphorus pentoxide in a desiccator to provide 2.09 g of 7-amino-3-iodomethyl-$\Delta^3$-cephem-4-carboxylic acid (yield: 67 %).

ii)

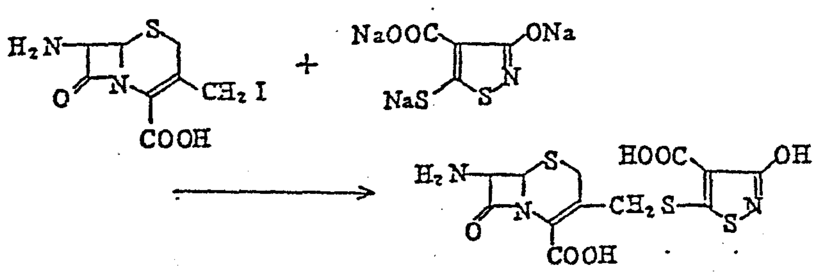

In 60 ml of water was suspended 3.4 g (0.01 mole) of 7-amino-3-iodomethyl-$\Delta^3$-cephem-4-carboxylic acid, and 0.84 g (0.01 mole) of sodium hydrogencarbonate was added thereto to form a solution. After adding thereto 2.67 g (0.011 mole) of 4-carboxy-3-hydroxy-5-mercapto-isothiazole tri-sodium salt, reaction proceeded for a period of 3 hours. After the reaction was over, the pH of the mixture was adjusted to 1.6 with 2N hydrochloric acid under cooling with ice-water. The mixture was stirred for 10 minutes under cooling with ice-water, and the precipitates thus formed were collected by filtration. The precipitates thus obtained were washed with 10 ml of cooled water, and dried to provide 2.9 g of 7-amino-3-[(4-carboxy-3-hydroxyisothiazol-5-yl)thiomethyl]-$\Delta^3$-cephem-4-carboxylic acid (yield: 74.5 %).

Melting point: 170°C (begin to color) ~ above 200°C (change to black-brown ).

NMR ($D_2O$+$NaHCO_3$)

δ(ppm); 3.58 (2H, q, 2-position: C$\underline{H}_2$)

3.98 (2H, q, -C$\underline{H}_2$-S-)

5.04 (1H, d, 6-position: CH)

5.41 (1H, q, 7-position : CH)

IR $\nu_{max}^{KBr}$ $cm^{-1}$; 1770 (lactam)

Example 1

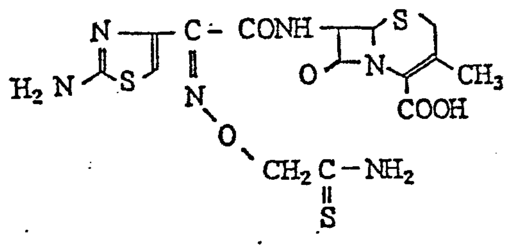

i) To 15 ml of dioxan were added 502 mg (1 milli mol) of (Z)-2-(thiocarbamoylmethoxyimino)-2-(2-tritylamino-4-thiazolyl-acetic acid obtained by Reference Example 1 iii), 152 mg (1.1 milli mol) of 1-hydroxybenzotriazol and 227 mg (1.1 milli mol) of dicyclohexylcarbodiimide, and the mixture was reacted for 1 hour at room temperature. After the reaction was over, dicyclohexylurea precipitated was removed by filtration to provide a dioxan solution of active ester. This solution was, at room temperature, added to a solution of 380 mg (1 milli mol) of 7-aminodeacetoxycephalosporanic acid benzhydryl ester in 15 ml of dioxane.

After the reaction had proceeded at room temperature for a period of 2.5 hours, dioxane was distilled off under reduced pressure to provide a caramel. The caramel was subjected to silica gel column chromatography, and the product was eluted with a mixture of n-hexane and ethyl acetate in a ratio of 1:1. The fractions containing the desired product were collected and the solvents were distilled off, and the residue was treated

with isopropyl ether to provide a powder of 650 mg of (Z)-3-methyl-7-[α-(2-amino-4-thiazolyl)-α-(thiocarbamoylmethoxyimino)-acetamido]-$\Delta^3$-cephem-4-carboxylic acid benzhydryl ester.

IR $\nu_{max}^{KBr}$ $cm^{-1}$ ;

3270, 2960, 2910, 1770, 1720, 1520, 1370, 1215, 1030, 695

NMR ($CDCl_3$)

2.08 (3H, s, $-CH_3$)

3.29 (2H, q, 2-position: $CH_2$)

5.03 (1H, d, 6-position: CH)

5.04 (2H, s, $-CH_2-$)

5.74 (1H, q, 7-position: CH)

6.90 (1H, s, 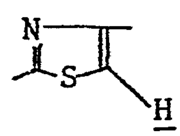 )

7.1 - 7.5 (25H, 5ϕ)

Mass spectl (FD-Mass)

$M^+$ m/e 864, 243

ii) To 640 mg of the compound obtained in Example 1(i) was added 2 ml of anisole to form a solution, and after adding thereto 4 ml of trifluoroacetic acid dropwise below 20°C, reaction proceeded for 1 hour at 19-21°C. After the reaction was over trifluoroacetic acid was distilled off under reduced pressure to provide an anisole solution. To the anisole solution was added 10 ml of isopropyl ether to form a powder. This powder

was dissolved in 7 ml of trifluoro acetic acid below 20°C, and after adding thereto 3.5 ml of water below 20°C, reaction proceeded at 19~21°C for 1 hour. After the reaction was over, the reaction mixture was concentrated under reduced pressure to remove trifluoroacetic acid and water. To the residue thus obtained was added 10 ml of ethyl alcohol to form a homogeneous solution, and some ethyl alcohol was distilled off to provide an oil, to which was added ethyl ether to provide a powder. This powder was collected by filtration and suspended in 5 ml of water. To the suspension was added 100 mg of sodium hydrogen carbonate to form a solution, and the solution was subjected to column chromatography on Diaion HP-20 (made by Mitsubishi Chemical Co., Japan), and the product was eluted first with 500 ml of water , and then with a mixture of water and methanol in a ratio of 9:1. The fractions containing desired product were collected, concentrated, and lyophilized to provide 25 mg of sodium (Z)-7-[α-(2-amino-4-thiazolyl)-α-(thiocarbamoylmethoxyimino)acetamide]-3-methyl-$\Delta^3$-cephem-4-carboxylate.

IR $\nu_{max}^{KBr}$ $cm^{-1}$ ;

3350-3450, 1750, 1550, 1580, 1530, 1400, 1030

NMR ($d_6$DMSO)

δ(ppm);

1.90 (3H, s, $CH_3$-)

3.34 (2H, q, 2-position: $CH_2$)

5.02 (1H, d, 6-position:CH)

5.05 (2H, s, $-CH_2-$)

5.56 (1H, s, 7-position: CH)

6.91 (1H, s, 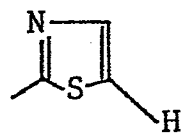 )

9.72 (1H, d, -<u>NH</u>- )

0148007

Example 2

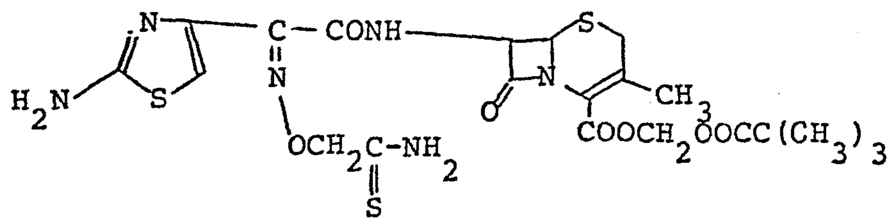

To 25 ml of dimethylformamide were added 657 mg of (z)-α-(thiocarbamoylmethoxyimino)-α-(2-amino-4-thiazolyl acetic acid, 341 mg of 1-hydroxybenzotriazol and 569 mg of dicyclohexylcarbodiimide, and the mixture was stirred at room temperature for 40 minutes. Dicyclohexylurea precipitated was removed by filtration to provide a dimethylformamide solution of active ester. This solution was added to 8 ml of a dimethylformamide solution containing 830 mg of pivaloyloxymethyl 7β-amino-3-methyl-$\Delta^3$-cephem-4-carboxylate obtained by Reference Example 2ii), then the mixture was stirred at room temperature overnight. To the reaction mixture was added 150 ml of ethyl acetate, and the mixture was washed six times with 50 ml of water and one time with 50 ml of a saturated aqueous sodium chloride solution. After the seperated organic layer was dried over magnesium sulfate, solvent was distilled off under reduced pressure to provide 1.65 g of a crude product. This crude product was subjected to silica gel column chromatography . The product was eluted with a mixture of ethyl acetate and benzene in a ratio of 2:1. The fractions containing the desired product were collected and concentrated to provide 1.15 g of pivaloyloxymethyl (Z)-7-[α-(2-amino-4-thiazoryl)-α-(thiocarbamoylmethoxyimino)acetamide]-3-methyl-$\Delta^3$-cephem-4-carboxylate.

IR $\nu_{max}^{KBr}$ $cm^{-1}$ ;

3275, 3175, 2960, 2920, 1770, 1740, 1660, 1620, 1530, 1370, 1020

NMR ($CDCl_3$ / TMS)

δ(ppm) 1.20 (9H, S, tBu)

2.16 (3H, S, $\underline{CH_3}$)

3.52 (2H, q, 2-position: $-\underline{CH_2}-$)

5.12 (2H, q, $-\underline{CH_2}\underset{\underset{S}{\|}}{C}NH_2$)

5.14 (1H, d, 6-position: $-\underline{CH}-$)

5.18 (2H, q, $COO\underline{CH_2}O-$)

and

(1H, q 7-position: $-C\underline{H}-$)

6.68 (1H, S, 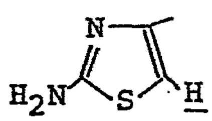 )

8.0 and 8.4 (each 2H, m, 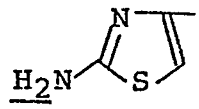 and 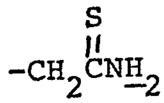 )

9.5 (1H, d, -CONH-)

FABMS; $(M+H)^+$ = 571

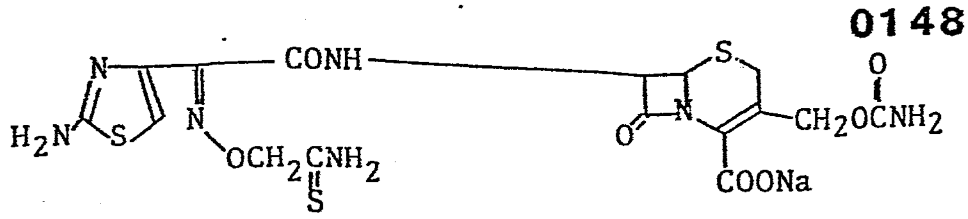

To 4 ml of dimethylformamide were added 260 mg of (Z)-α-(thiocarbamoylmethoxyimino)-α-(2-amino-4-thiazolyl)acetic acid, 135 mg of 1-hydroxybenzotriazole and 225 mg of dicyclohexylcarbodiimide, and the mixture was stirred for 1 hour at room temperature. Dicyclohexylurea precipitated was removed by filtration to provide a dimethylformamide solution of active ester. This solution was added to a suspension of 387 mg of 7-amino-3-carbamoyloxymethyl-$\Delta^3$-cephemcarboxylic acid in 4 ml of dimethylformamide, and the mixture was stirred overnight. The reaction solution was poured into 100 ml of water, and after adjusting the pH of the mixture to 8 by adding thereto 4 ml of saturated sodium hydrogencarbonate, the aqueous layer was washed 3 times with 25 ml of ethyl acetate. The pH of the aqueous layers separated was adjusted to 2 with 1N-hydrochloric acid. Insoluble solids thus precipitated were collected by filtration. The insoluble solids collected were dissolved in dilute aqueous sodium hydrogencarbonate solution, and the solution was subjected to column chromatography on Diaion HP-20 and the product was eluted first with water and then with mixtures of water and methanol of successively changing mixing ratio (water:methanol from 9:1 to 8:2). The fractions containing the desired product were concentrated and lyophilized provide 230 mg of sodium salt of (Z)-7-[α-(2-amino-4-thiazolyl)-α-(thiocarbamoylmethoxyimino))acetamido]-3-carbamoyloxymethyl-$\Delta^3$-cephem-4-carboxylic acid.

IR $\nu_{max}^{KBr}$ $cm^{-1}$;

3350, 1755, 1600, 1530,
1390, 1320, 1025

NMR (DMSO-$d^6$-TMS)

δ(ppm);

| | |
|---|---|
| 3.36 | (2H, q, 2: $-CH_2-$) |
| 4,76 | (4H, bs, $-C\underline{H}_2O\underset{\underset{O}{\|}}{C}NH_2$ and $-C\underline{H}_2\underset{\underset{S}{\|}}{C}NH_2$) |
| 5.02 | (1H, d, 6: -CH-) |
| 5.64 | (1H, d, 7: -CH-) |
| 5.86 | (1H, s, 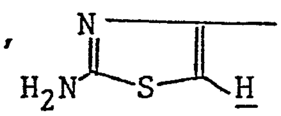 ) |

FABMS; $(M+Na)^+$ =538

Example 4

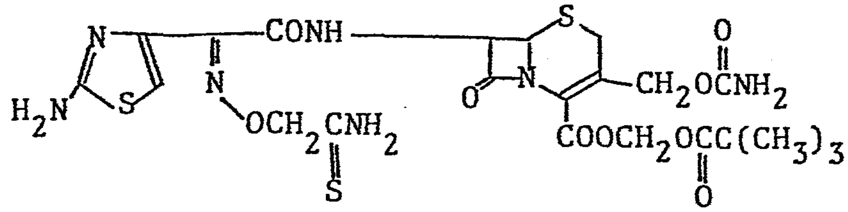

To 4 ml of dimethylformamide was added 100 mg of sodium salt of (Z)-7-[α-(2-amino-4-thiazolyl)-α-(thiocarbamoylmethoxyimino)acetamido]-3-carbamoyloxymethyl-$\Delta^3$-cephem-4-carboxylic acid otained by Example 3, and the mixture was cooled to 0°C. To the mixture was added 68 mg of pivaloyloxymethyliodide and the mixture was stirred for 1 hour at 0-3°C. To the reaction mixture was added 40 ml of ethyl acetate, and the mixture was washed three times with 10 ml of water, and then once with 10 ml of a saturated aqueous sodium chloride solution. The organic layer separated was dried over magnesium sulfate, and ethyl acetate was distilled off under reduced pressure. The residue was treated with ether to form a powder, and the powder was collected by filtration and dried to provide 46 mg of pivaloyloxymethyl (Z)-7-[α-(2-amino-4-thiazolyl)-α-(thiocarbamoylmethoxyimino)acetamido]-3-carbamoyloxymethyl-$\Delta^3$-cephem-4-carboxylate.

IR $\nu_{max}^{KBr}$ $cm^{-1}$;

3300, 3275, 3050, 1770, 1730, 1660, 1600, 1520, 1380, 1320, 1220, 1020.

NMR (DMSO-$d^6$ -TMS)

δ (ppm): 1.20 (9H, s, tert-Butyl)

3.36 (2H, q, 2-position: $-C\underline{H}_2-$)

4.7 (2H, s, $-C\underline{H}_2O\underset{\underset{O}{\|}}{C}NH_2$ and

2H, q, $-C\underline{H}_2\underset{\underset{S}{\|}}{C}NH_2$)

5.18 (1H, d, 6-position: $-C\underline{H}-$)

5.80 (2H, q, $-COOC\underline{H}_2O-$ and

1H, q, 7-position: $-C\underline{H}-$)

6.84 (1H, s, 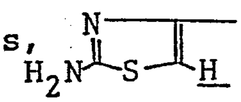 )

8.68, 10.12 (each 1H, $-CH_2\underset{\underset{S}{\|}}{C}N\underline{H}_2$)

9.72 (1H, d, $-CON\underline{H}-$)

Example 5

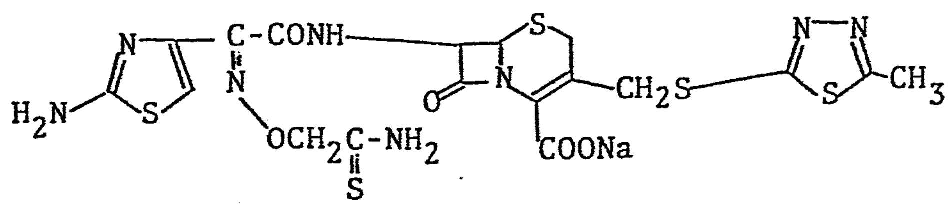

To 4 ml of dimethylformamide were added 260 mg of (Z)-α-(thiocarbamoylmethoxyimino)-α-(2-amino-4-thiazolyl)acetic acid, 138 mg of 1-hydroxybenzotriazole and 207 mg of dicyclohexylcarbodiimide, and the mixture was stirred for 1 hour at room temperature. Dicyclohexylurea precipitated was removed by filtration to provide a dimethylformamide solution of active ester.

In 4 ml of water was suspended 344 mg of 7β-amino-3-[(5-methyl-1,3,4-thiadiazol-2-yl)thiomethyl]-$\Delta^3$-cephem-4-carboxylic acid, and 84 mg of sodium hydrogencarbonate was added portionwise thereto to provide a solution. To the formed pale brown solution was added portionwise the foregoing dimethylformamide solution of active ester. After the addition, the mixture was stirred for 1 hour at room temperature, and poured into 100 ml of water. The aqueous layer was washed with 50 ml of ethyl acetate, and the aquous layer separated was subjected to column chromatography on Diaion HP-20, and the product was eluted with water and then with mixtures of water and methanol of successively changing mixing ratio (volume ratio of water : methanol from 9:1 to 7:3). The fractions containing the desired product were collected, concentrated, and

lyophilized to provide 221 mg of (Z)-7-[α-(2-amino-4-thiazolyl)-α-(thiocarbamoylmethoxyimino))acetamido]-3-[(5-methyl-1,3,4-thiadiazol-2-yl)thiomethyl]-$\Delta^3$-cephem-4-carboxylic acid sodium salt.

IR $\nu_{max}^{KBr}$ $cm^{-1}$;

3400-3350, 1755, 1600, 1530, 1380, 1190, 1030.

NMR ($d_6$-DMSO)

δ(ppm); 2.64 (3H, s, $C\underline{H}_3$)

3.52 (2H, q, 2-position: $C\underline{H}_2$)

4,43 (2H, q, 3-position: $-C\underline{H}_2-$)

4.47 (2H, s, $-C\underline{H}_2-$)

5.04 (1H, d, 6-position: C$\underline{H}$)

5.64 (1H, q, 7-position: C$\underline{H}$)

6.86 (1H, s, 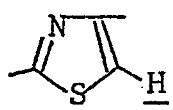 )

7.31 (2H, s, $\underline{H}_2N-$)

8.78, 10.9 (each 1H, each s, $-\underset{\underset{S}{\|}}{C}-N\underline{H}_2$)

9.72 (1H, d, -CON$\underline{H}$-)

FABMAS; $(M+1)^+=609$, $(M+Na)^+=631$

Example 6

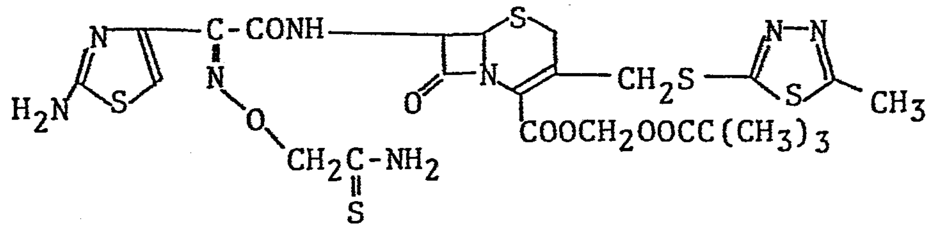

To 5 ml of dimethylformamide was added 100 mg of (Z)-7-{α-(2-amino-4-thiazolyl)-α-(thiocarbamoylmethoxy imino)acetamido}-3-[(5-methyl-1,3,4-thiadiazol-2-yl-thiomethyl]-$\Delta^3$-cephem-4-carboxylic acid sodium salt, and the mixture was cooled to 0°C. To the mixture was added a solution of 45 mg of pivaloyloxy)methyl iodide in 1 ml of dimethylformamide at -1 to 0°C. After stirring the mixture for 90 minutes at 0°C, the mixture was poured into 50 ml of ice-water. The mixture was extracted with 50 ml of ethyl acetate and then with 25 ml of ethyl acetate. The organic layer separated was washed with 5 ml of saturated sodium hydrogencarbonate and twice with 10 ml of water, and then with 10 ml of a saturated sodium chloride solution. The organic layer separated was dried over anhydrous sodium sulfate, and ethyl acetate was distilled under reduced pressure.

The residue was treated with 30 ml of ether-n-hexane (volume ratio of 1:1) to provide a powder.

The powder was collected by filtration and dried to provide 70 mg of pivaloyloxymethyl (Z)-7-[α-(2-amino-4-thiazolyl)-α-(thiocarbamoylmethoxyimino)acetamido]-3-[(5-methyl-1,3,4-thiadiazol-2-yl)thiomethyl]-$\Delta^3$-cephem-4-carboxylate.

IR $\nu_{max}^{KBr}$ $cm^{-1}$

3270, 3160, 2950, 2910, 2850, 1770, 1740, 1660, 1610, 1525, 1370, 1105, 1020, 980.

NMR ($d_6$-DMSO )

δ(ppm);

1.14 (9H, S, t-Bu- )

2.67 (3H, S, $\underline{CH_3}$- )

3.74 (2H, q, 2-position; $\underline{CH_2}$ )

4.36 (2H, q, 3-position; -$\underline{CH_2}$- )

4.74 (2H, S, -$\underline{CH_2}$- )

5.22 (1H, d, 6-position; $\underline{CH}$ )

5.7 - 6.0 (3H, 7-position; $\underline{CH}$, -COO$\underline{CH_2}$- )

6.88 (1H, S, 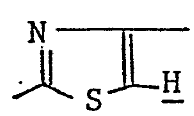 )

7.30 (2H, S, -$\underline{NH_2}$ )

8.75, 10.1 (each 1H, each S, 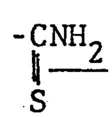 )

9.78 (1H, d, -CO$\underline{NH}$- )

FABMAS.; $(M+1)^+$=701

Example 7

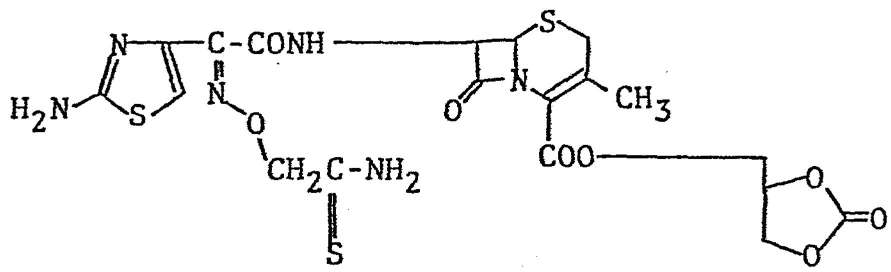

In 5 ml of dimethylformamide were dissolved 112 mg of (Z)-α-(thiocarbamoylmethoxyimino)-α-(2-amino-4-thiazolyl)acetic acid and 58 mg of 1-hydroxybenzotriazole and after adding thereto 97 mg of dicyclohexylcarbodiimide, the mixture was stirred for 1 hour. Dicyclohexylurea precipitated was removed by filtration to provide a dimethylformamide solution of active ester. The dimethylformamide solution was added to a solution of 123 mg of 2-oxo-1,3-dioxolan-4-ylmethyl 7-amino-3-methyl-$\Delta^3$-cephem-4-carboxylate trifluoroacetic acid salt (obtained by Reference Example 3 ii)) in 1 ml of dimethylformamide, and the mixture was stirred overnight at room temperature. To the reaction mixture was added 30 ml of ethyl acetate, and the mixture was washed three times with 30 ml of water and once with 30 ml of a saturated aquous sodium chloride solution, and dried over magnesium sulfate. Solvent was distilled away under reduced pressure, and the residue was subjected to silica gel column chromatography. The product was eluted with ethyl acetate, and the fractions cotaining the desired product were collected, and concentrated

to provide 136 mg of 2-oxo-1,3-dioxolan-4-ylmethyl (Z)-7-[α-(2-amino-4-thiazolyl)-α-(thiocarbomethoxyimino)-acetamido]-3-methyl-$\Delta^3$-cephem-4-carboxylate.

IR $\nu_{max}^{KBr}$ $cm^{-1}$;

3400, 2900, 1770, 1720, 1620, 1520, 1380, 1300, 1240, 1220, 1060.

NMR (DMSO-$d^6$/TMS)

δ(ppm); 2.04 (3H, s, $-C\underline{H}_3$)

3.54 (2H, q, 2-position: $-C\underline{H}_2-$)

4.2-4.6 (4H, m, 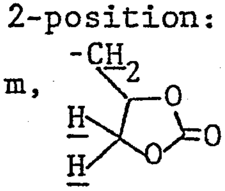 )

4.74 (2H, s, $-C\underline{H}_2\overset{S}{\overset{\|}{C}}NH_2$)

4.9-5.3 (1H, m, 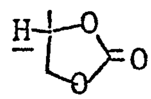 )

5.16 (1H, d, 6-position: $-C\underline{H}-$)

5.76 (1H, q, 7-position: $-C\underline{H}-$)

6.90 (1H, s, 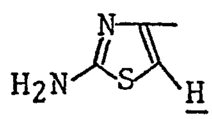 )

7.30 (2H, bs, 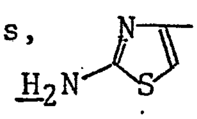 )

8.76, 10.16 (each 1H, bs, $-CH_2\overset{S}{\overset{\|}{C}}N\underline{H}_2$)

9.80 (1H, d, $-CON\underline{H}-$)

FABMS : $(M+H)^+$=557

Example 8

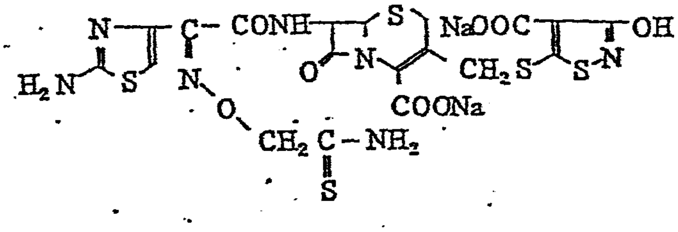

i) In 15 ml of dimethylformamide was dissolved 753 mg of (Z)-α-(2-tritylamino-4-thiazolyl)-α-thiocarbamoylmethoxyiminoacetic acid and after adding thereto 200 mg of 1-hydroxybenzotriazole and 310 mg of dicyclohexylcarbodiimide, reaction proceeded for 1 hour at room temperature. After the reaction was complete, dicyclohexylurea precipitated was removed by filtration to provide a dimethylformamide solution containing active ester.

In 6 ml of water was suspended 584 mg of 7-amino-3-[(4-carboxy-3-hydroxy-5-isothiazolyl)thiomethyl]-$\Delta^3$-cephem-4-carboxylic acid obtained by Reference Example 4 ii), and 255 mg of sodium hydrogencarbonate was added portionwise thereto to provide a solution. To the clear brown solution thus obtained was added the foregoing dimethylformamide solution of active ester, and the mixture was stirred for 4 hours at room temperature. The reaction solution was poured into 20 ml of water and 5 ml of a saturated aqueous sodium hydrogencarbonate solution, and the mixture was extracted by washing twice with 50 ml of ethyl acetate.

To the aqueous layer was added 15 ml of 2N-hydrochloric acid, and the mixture was extracted with 100 ml of methylethylketone and then with 50 ml of methylethylketone. Insoluble matters precipitated were removed by filtration. The methylethylketone extract was washed twice with 30 ml of water and then with 30 ml of saturated aquous sodium chloride, and dried over anhydrous magnesium sulfate. Methylethylketone was removed by distillation under reduced pressure to provide a caramel. The caramel was subjected to silica gel column chromatography and the product was eluted with a mixture of chloroform-isopropylalcohol-formic acid (volume ratio, 80:20:2). The fractions containing the desired product were collected, and solvent was removed by distillation. To the residue was added isopropylether to form a powder, which was collected to provide 330 mg of (Z)-3-[(4-carboxy-3-hydroxy-5-isothiazolyl)thiomethyl]-7-[α-(thiocarbamoylmethoxyimino)-α-(2-tritylamino-4-thiazolyl)acetamido]-$\Delta^3$-cephem-4-carboxylic acid.

IR $\nu_{max}^{KBr}$ $cm^{-1}$;

3280-3260, 3190-3160, 2950, 1770, 1610, 1520, 1440, 1240, 1030, 995, 750, 695

NMR ($d_6$-DMSO)

δ(ppm); 4.12 (2H, q, 3-position: $-CH_2-$)
4.71 (2H, s, $-OC\underline{H}_2CS-$)
5.15 (1H, d, 6-position: CH)

5.71 (1H, q, 7-position: CH)

6.79 (1H, s, 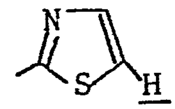 )

7.0-7.4 (15H, 3Φ )

8.90 and 10.1 (2H, 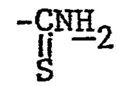

9.64 (1H, d, -CONH)

ii) To 7 ml of trifluoroacetic acid cooled to 5°C was added 300 mg of the compound obtained by Example 8i). To the formed solution was added 3.5 ml of water dropwise below 20°C, and reaction proceeded for 1 hour at 19-21°C. After the reaction was complete, the mixture was concentrated under reduced pressure to remove trifluoroacetic acid and water. To the formed residue was added 10 ml of ethylalcohol to form a uniform solution. Some alcohol was distilled away to form an oil. To the oil was added ethylether to form a powder, and 230 mg of the crude product powder was obtained.

This crude product was suspended in 5 ml of water, and 100 mg of sodium hydrogencarbonate was added thereto to form a solution. The solution was subjected to column chromatography on Diaion HP-20 (Mitshubishi Chemical Co., Ltd., Japan), and the product was eluted with mixtures of water-methanol of successively changing mixing ratio (water:methanol from 1:0 to 8:2). The fractions containing the desired product were collected, concentrated, and lyophilized to provide 35 mg of (Z)-7-[α-(2-amino-4-thiazolyl)-α-(thiocarbamoylmethoxyimino)acetamido]-3-[(4-carboxylate-3-hydroxy-5-isothiazolyl)thiomethyl]-$\Delta^3$-cephem-4-carboxylate.

IR $\nu_{max}^{KBr}$ $cm^{-1}$;

3380-3330, 1760, 1610, 1525, 1380-1370, 1020, 815, 760.

NMR ($d_6$-DMSO)

δ(ppm); 4.76 (2H, s, -OC$\underline{H}_2$CS-)

5.06 (1H, d, 6-position: CH)

5.62 (1H, q, 7-position: CH)

6.87 (1H, s, 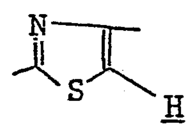 )

9.75 (1H, d, -CON$\underline{H}$-)

Claims:

1. A compoud of general formula (I) or salt thereof:

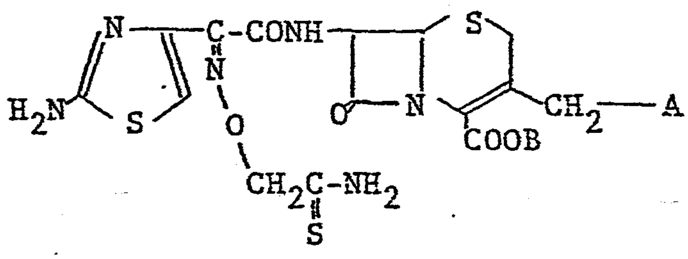

(I)

wherein A represents a hydrogen atom, a carbamoyloxy group, (4-carboxy-3-hydroxyisothiazol-5-yl)thio group (that is, 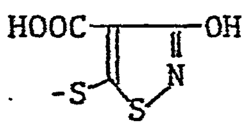 ),

or a group shown by the formula:

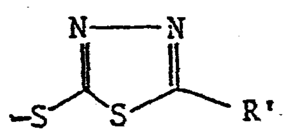 (where R' is a $C_1$-$C_4$ alkyl group);

and B represents a hydrogen atom, 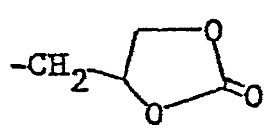 ,

$-CH_2OOC—C(R^2)_3$ (where $R^2$ is a $C_1$-$C_4$ alkyl group), or a cation forming a pharmaceutically acceptable carboxylate salt.

2. A compound of claim 1 wherein B represents a hydrogen atom, an alkali metal atom, pivaloyloxymethyl or 2-oxo-1,3-dioxolan-4-ylmethyl.

3. 7-[α-(2-amino-4-thiazolyl)-α-(thiocarbamoylmethoxyimino)acetamido]-3-methyl-$\Delta^3$-cephem-4-carboxylic acid according to claim 1.

4. A syn-form compound according to claim 1 or 2 or 3.

5. A pharmaceutical composition, preferably for oral administration, containing as an active ingredient thereof a compound according to any preceding claim.

6. A method of producing antibacterial activity by administering, preferably orally, a compound according to any of claims 1 to 4.

7. A process for producing a compound according to claim 1 which is characterized by reacting a compound of the formula :

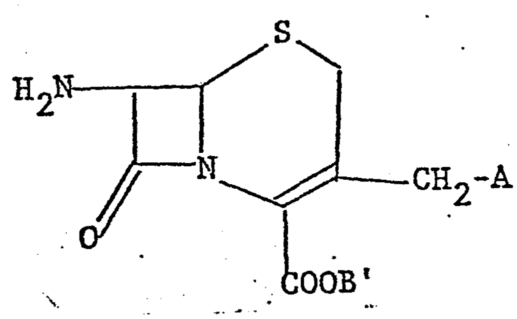

wherein A is as defined above, and B' is a B group as defined

above or a protective group for the carboxyl group, with a compound of the formula :

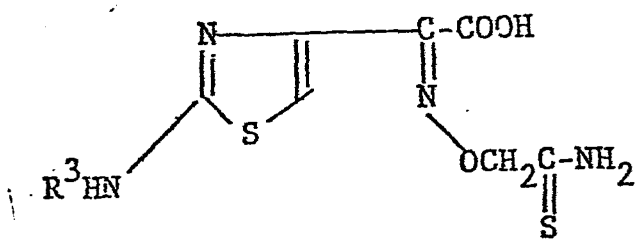

wherein $R^3$ represents a hydrogen atom or a protective group for the amino group, or reactive derivative thereof; and if necessary, removing the protective group(s).

8. A process of producing a compound of the formula :

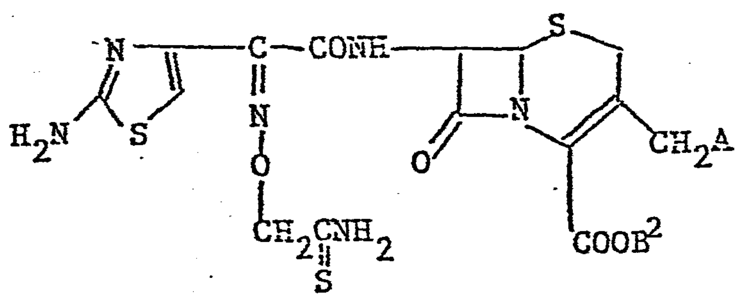

wherein A is as defined above and $B^2$ represents $-CH_2OOCC(R^2)_3$ or 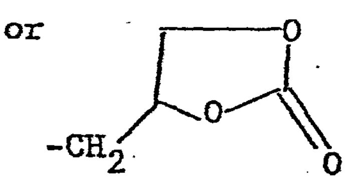 (wherein $R^2$ represents a $C_1$-$C_4$ alkyl group) which is characterized by esterifying a compound of the formula :

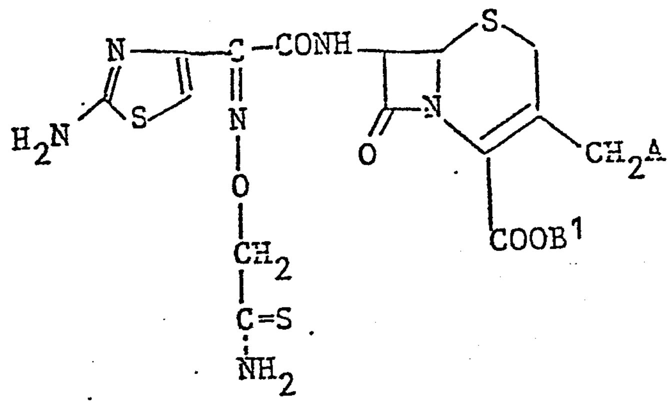

wherein A is as defined above, and $B^1$ represents a hydrogen atom or a cation.